# EUROPEAN PATENT SPECIFICATION

(11) **EP 1 435 937 B1**
(45) Date of publication and mention of the grant of the patent: **15.06.2005**
(21) Application number: 02767718.6
(22) Date of filing: 16.10.2002
(51) Int. Cl.: A61K 31/167, A61K 31/165, A61P 25/04

(54) **COMPOSITION COMPRISING PARACETAMOL AND A BITTERNESS MASKING COMPONENT**
ZUBEREITUNG ENTHALTEND PARACETAMOL UND EIN GESCHMACKSMASKIERUNGSMITTEL
COMPOSITION CONTENANT DU PARACETAMOL ET UN CONSTITUANT MASQUANT L'AMERTUME

(30) Priority: 18.10.2001 GB 0125022
(43) Date of publication of application: 14.07.2004
(73) Proprietor: Reckitt Benckiser Healthcare (UK) Limited, Slough, Berkshire SL1 3UH (GB)
(72) Inventor: LINDLEY, Michael George, Reading Berkshire RG6 6LA (GB)
(74) Representative: Hall, Marina
(86) International application number: PCT/GB2002/004653
(87) International publication number: WO 2003/032973

(56) References cited:
- EP-A- 0 127 381
- WO-A-01/37816
- WO-A-95/00133
- DATABASE WPI , 1997 Derwent Publications Ltd., London, GB; AN 048244 XP002228416 KOMATSU: "Preventing Whisker generation. " & JP 08 301764 A (NISSUI PHARMA), 10 May 1995 (1995-05-10)

## Description

The present invention relates to pharmaceutical compositions comprising paracetamol.

Paracetamol is a well known compound having pharmaceutical properties. One of its best known modes of action is as an analgesic. It is also known that paracetamol has an extremely bitter taste which can make it particularly unpleasant to take orally.

Paracetamol based compositions are often used as "cold remedies". Such compositions may not cure the underlying condition, but may treat or alleviate the symptoms of colds or influenza, including headaches, fever, rhinitis and general aches and pains. An example of such a composition is "Lemsip" (trade mark) which is a lemon-flavoured powdered composition intended to be diluted with warm or hot water before use. Such a composition is formulated with sweeteners such that the bitter taste of the paracetamol is masked. In order to mask the bitter taste to an acceptable extent when the composition is diluted with about 150 ml water, a mixture of no less than three sweeteners has been found to be necessary. These sweeteners are sugar (sucrose), aspartame and saccharin.

However, when the composition is diluted with lesser amounts of water, for example about 20 to 100 ml water, the sweeteners present in the composition make the composition overpoweringly sweet. Reducing the amount of sweeteners can overcome this problem, but then the bitter taste of the paracetamol is insufficiently masked. Thus ready-to-use formulations containing lesser amounts of water cannot be satisfactorily prepared.

We have found that the bitter taste of paracetamol can be masked by using a combination of components.

Accordingly the present invention provides a pharmaceutical composition comprising paracetamol and a masking component which reduces or masks the bitter taste thereof which comprises at least two members chosen from the following groups i to vi:
i. pharmacologically acceptable magnesium salts, magnesium hydroxide and/or magnesium oxide;
ii. gingerin and/or ginger oil;
iii. a delayed onset long lasting sweetener;
iv. soluble starch in a weight ratio of at least 1:15 with respect to the paracetamol;
v. an edible emulsifier having an HLB value of greater than 10; and
vi. glycine.
   It has been found by the inventors that it is not possible to mask the taste or bitterness of paracetamol effectively, especially in liquid formulations comprising less than 100 ml water, by using a single masking agent. Instead it has been found that a combination of different masking agents is required. It has been found that even if one component is used in a concentrate such that it has its maximum effect in reducing the bitterness, the use of further components can surprisingly still reduce the bitterness taste further.
   Component i is a pharmacologically acceptable magnesium salt, magnesium oxide and/or magnesium hydroxide. One or more magnesium salts, oxide or hydroxide may be used. A pharmacologically acceptable salt is one which can safely be ingested in the amounts present in a dose of the composition. Desirably the counter anion does not have an adverse masking effect. Accordingly it is preferred to avoid the use of magnesium sulfate, although it may be used if the other components of the composition provide an adequate masking effect. For example a mixture of magnesium chloride and magnesium sulfate may be used. It is also preferred to avoid using counter anions which have their own taste or which provide an undesired sensation such as bitterness or saltiness. Examples of salts which are advantageously used are magnesium chloride, gluconate, hydroxide, carbonate and/or bicarbonate.
   Component ii is gingerin (also known as ginger oleoresin) or ginger oil (also known as gingerol). These components are extracted from ginger, and are commercially available from, for example, Sigma-Aldrich Company Ltd. Gingerin and gingerol may also be used together as a mixture.
   Component iii is a delayed onset long lasting sweetener. The term "delayed onset long lasting sweetener" is a term of art and includes sweeteners which have a long-lasting action but which may not necessarily provide an initial burst of sweetness. Examples of delayed onset long lasting sweeteners are Neohesperidine DC, Thaumatin and Glycyrrhizin, of which Thaumatin is preferred. Thaumatin is available, for example, under the trade mark Talin.
   Component iv is soluble starch (also known as instant starch). Soluble starch is widely commercially available. An example of a suitable soluble starch is Ultratex 4 obtainable from National Starch.
   Component v is an edible emulsifier. Such compounds are often used in, for example, the food manufacturing industry to emulsify ingredients. Examples of edible emulsifiers are sugar esters, for example esters of sucrose, glucose, galactose or fructose, in particular with fatty acids (for example containing from 10 to 24 carbon atoms) and vegetable oils such as soya oil. The edible emulsifiers have a high HLB value of greater than 10, preferably at least 12, more preferably at least 14, and generally yield stable milky dispersions to clear solutions in water. Such emulsifiers are obtainable from, for example, Sistema B.V. Netherlands. Other useful emulsifiers are, for example, lecithin, glycerol mono stearate, diglycerin and the Polysorbate series such as Polysorbate 80.
   Component vi is glycine. Glycine is widely available commercially, for example from Merck Ltd.
   Optionally, but desirably, the masking component may also comprise:
vii. a pharmacologically acceptable sodium salt.

Component vii is a pharmacologically acceptable sodium salt. One or more sodium salts may be used. A pharmacologically acceptable salt is one which can safely be ingested in the amounts present in a dose of the composition. Desirably the counter anion does not have an adverse masking effect. Accordingly it is preferred to avoid the use of sodium sulfate, although it may be used if the other components of the composition provide an adequate masking effect. It is also preferred to avoid using counter anions which have their own taste or which provide an undesired sensation such as bitterness or saltiness. Examples of salts which are advantageously used are sodium acetate, carbonate, bicarbonate, citrate, gluconate, malate, lactate and chloride, propionate, oxalate, malonate, succinate, furmanate, tartrate and benzoate.

The masking component comprises a mixture of the above individual components in amounts such that the bitter taste of paracetamol can be masked. The amounts of the individual components, and the total amount of the components, can be determined easily by one skilled in the art using appropriate techniques.

For example, a base composition comprising 650 mg paracetamol and 50 mg ascorbic acid in 40 ml water may be prepared, and, if desired, appropriate amounts of base components such as sweeteners and lemon flavour added. The base composition is used as a control. In order to test the effectiveness of a particular component, a measured amount of that component is added to the base formulation to prepare a first test formulation. Further test formulations are then prepared in a similar way but containing different amounts of the component to be tested. The test compositions are then assessed for masking of the bitter taste of the paracetamol, as compared with the control formulation, by a panel of testers, for example 3 or 4 testers. From this test, appropriate amounts of individual components can be determined. The test can be modified appropriately for testing two or more compounds by first deciding on an appropriate amount of the first compound, and then repeating the test with the same amount of the first compound in the control formulation and all of the test formulations. The amounts of each components will depend on various factors, including the possible presence of other masking components and the intended volume of dilution of the composition.

The magnesium salt, hydroxide or oxide (i) is desirably present in a molar ratio of up to 0.01:1, calculated as magnesium atoms: paracetamol, preferably from 0.0001:1 to 0.01:1, more preferably from 0.001:1 to 0.01:1, most preferably from 0.001:1 to 0.005:1.

The gingerin and/or gingerol (ii) is generally used in an amount such that if it does not itself add any taste to the composition, although it is possible to use it in such an amount that the composition has a ginger taste if desired. Generally the gingerin and/or gingerol is used in a weight ratio of up to 1:10,000 gingerin and/or gingerol: paracetamol, preferably from 1:30,000 to 1:10,000, more preferably from 1:20,000 to 1:10,000.

The delayed onset long lasting sweetener (iii) is generally used in a weight ratio of up to 1:100 with respect to the paracetamol, especially from 1:20,000 to 1:100, preferably from 1:10,000 to 1:100, and more preferably from 1:1,000 to 1:200.

The soluble starch (iv) is used in a weight ratio of at least 1:15 with respect to the paracetamol and generally used in a weight ratio of up to 1:1 with respect to the paracetamol, especially from 1:10 to 1:1 and more especially from 1:10 to 1:2.

The edible emulsifier (v) is generally used in a weight ratio of up to 2:1 with respect to the paracetamol, especially from 1:40 to 2:1, preferably from 1:10 to 2:1, more preferably from 1:5 to 1:1, and even more preferably from 1:4 to 1:1.

The glycine (vi) is generally used in a molar ratio of up to 2:1 with respect to the paracetamol, especially from 1:4 to 2:1, preferably from 1:4 to 1:1.

The sodium salt (vii) in general is used in an amount such that it does not itself add any taste to the composition, especially if sodium chloride is used. Desirably the sodium salt (vii) is present in a molar ratio of up to 1:1 calculated as sodium atoms: paracetamol, preferably from 0.01:1 to 1:1, more preferably from 0.1:1 to 1:1 and most preferably from 0.3:1 to 0.7:1.

The amount of the masking component containing the individual masking compounds which is used will, of course, vary depending on the individual compounds chosen. As a guide, however, it is generally used in an amount of from 0.00001g to 1g, preferably from 0.00006g to 0.6g, per gram of paracetamol.

The masking component used in the present invention need only contain two members selected from groups i to vi and optionally vii. By this we mean that one member may be chosen from one of the groups and the other is chosen from any of the other groups, but not the same group. It is, however, possible for more than one compound to be chosen from any group, so long as components from at least two different groups are chosen. A single compound may act simultaneously as a compound from at least two different groups, for example a mixed salt containing both sodium and magnesium ions.

If members from two different groups are chosen, they may be chosen from, for example, groups i and ii; i and iii; i and iv; i and v; i and vi; ii and iii; ii and iv; ii and v; ii and vi; iii and iv; iii and v; iii and vi; iv and v; iv and vi; and v and vi.

If members from three different groups are chosen, they may be chosen from, for example, groups i, ii and iii; i, ii and iv; i, ii and v; i, ii and vi; i, ii and vii; i, iii and iv; i, iii and v; i, iii and vi; i, iii and vii; i, iv and v; i, iv and vi; i, iv and vii; i, v and vi; i, v and vii; i, vi and vii; ii, iii and iv; ii, iii and v; ii, iii and vi; ii, iii and vii; ii, iv and v; ii, iv and vi; ii, iv and vii; ii, v and vi; ii, v and vii; ii, vi and vii; iii, iv and v; iii, iv and vi; iii, iv and vii; iii, v and vi; iii, v and vii; iii, vi and vii; iv, v and vi; iv, v and vii; iv, vi and vii; and v, vi and vii.

If members from four different groups are chosen, they may be chosen from, for example, groups i, ii, iii and iv; i, ii, iii and v; i, ii, iii and vi; i, ii, iii and vii; i, ii, iv and v; i, ii, iv and vi; i, ii, iv and vii; i, ii, v and vi; i, ii, v and vii; i, ii, vi and vii; i, iii, iv and v; i, iii, iv and vi; i, iii, iv and vii; i, iii, v and vi; i, iii, v and vii; i, iii, vi and vii; i, iv, v and vi; i, iv, v and vii; i, iv, vi and vii; i, v, vi and vii; ii, iii, iv and v; ii, iii, iv and vi, ii, iii, iv and vii; ii, iii, v and vi; ii, iii, v and vii; ii, iii, vi and vii; iii, iv, v and vi; iii, iv, v and vii; iii, iv, vi and vii; iii, v, vi and vii; and iv, v, vi and vii.

If members from five different groups are chosen, they may be chosen from, for example, groups i, ii, iii, iv and v; i, ii, iii, iv and vi; i, ii, iii, iv and vii; i, ii, iii, v and vi; i, ii, iii, v and vii; i, ii, iv, v and vi; i, ii, iv, v and vii; i, ii, iv, vi and vii; i, ii, v, vi and vii; i, iii, iv, v and vi; i, iii, iv, v and vii; i, iii, iv, vi and vii; i, iii, v, vi and vii; i, iv, v, vi and vii; ii, iii, iv, v and vi; ii, iii, iv, v and vii; ii, iii, iv, vi and vii; ii, iii, v, vi and vii; ii, iv, v, vi and vii; and iii, iv, v, vi and vii.

If members from six different groups are chosen, they may be chosen from, for example, groups i, ii, iii, iv, v and vi; i, ii, iii, iv, v and vii; i, ii, iii, iv, vi and vii; i, ii, iii, v, vi and vii; i, ii, iv, v, vi and vii; i, iii, iv, v, vi and vii; and ii, iii, iv, v, vi and vii.

It is also possible to have members chosen from all seven groups, namely groups i, ii, iii, iv, v, vi and vii.

The composition may also comprise at least one other component if desired. Thus it may, for example, comprise other active compounds such as ibuprofen, naproxen, diclofenac, ketoprofen or pseudoephedrine hydrochloride; a flavouring agent such as lemon, berry, tutti frutti, pineapple, orange, menthol or mint flavour; dried fruit; sweeteners such as saccharin or the sodium salt thereof, a sugar such as sucrose or glucose or aspartame; components producing effervescence such as a mixture of an acid such as citric acid and a carbonate or bicarbonate such as sodium or potassium bicarbonate; colouring agents; preservatives; filler, pH adjusting agents such as acids, for example citric acid, and bases; pH buffers; solvents; and fillers. It should be understood that any of the additional components may also act as a component selected from groups i to vii as defined above. Thus, for example, sodium bicarbonate can act both as a part of the effervescent producing component and as a member of group vii.

The composition can be provided in any suitable form. Thus, for example, it may be in the form of a solid such as a powder, granulate or tablet. The solid form can simply be dissolved in water, generally warm or hot water, before use. Desirably the composition is dissolved in less than 100 ml water, preferably from 30 to 60 ml water, most preferably from 40 to 50 ml water.

Preferably, however, the composition is sold in the form of a ready-to-use formulation which is already in aqueous form by dissolving or dispensing the abovementioned components in water. Desirably the composition has a total volume of less than 100 ml, preferably less than 50 ml, more preferably from 30 to 50 ml and most preferably about 40 ml. The aqueous composition may be sold in a package which is opened by a consumer shortly before use, and the composition heated, if desired, by the consumer. Preferably, however, the composition is sold in a self-heating container which heats the composition to a desired temperature before use and without the consumer having to take any action apart from opening the container. Such containers may be obtained from, for example, Thermotic Developments Ltd, UK; Chian & Forti S.p.A., Italy; and Ontro Inc, USA.

In order to prepare the compositions of the invention, the individual components may simply be mixed. The order of mixing is not critical. The masking component may, for example, be prepared separately and then added to the paracetamol, optionally in the presence of water and/or other components. It is also possible, for example, to add the components of the masking component individually.

The composition may be administered to a subject in need thereof or liable to be in need therefore in any effective amount. Suitable doses of paracetamol are well known, for example, from 0.5g to 1g per dose for an adult every 4 to 6 hours.

The composition may be sold in bulk form. Desirably, however, the composition is in unit dosage form.

The present invention further provides the use of a masking component as defined above to reduce or mask the bitter taste of paracetamol.

The present invention additionally provides the use of a masking component as defined above in the manufacture of a medicament comprising paracetamol for the treatment of the symptoms of colds and influenza in humans.

Gingerin, ginger oil and starch have not previously been known to act as masking components for the bitterness of paracetamol. Starch has not previously been known to act as masking component for bitterness of any component.

Accordingly the present invention also provides the use of gingerin, ginger oil, soluble starch or a mixture thereof to reduce or mask or mask the bitter taste of paracetamol.

The present invention is now further described in the following Examples.

### Examples

### Example 1

A composition was prepared by mixing together the following components in the amounts indicated:

| | |
|---|---|
| Paracetamol | 0.6500 g |
| Ascorbic acid | 0.0500 g |
| Caster sugar | 3.5000 g |
| Citric acid | 0.1868 g |
| Sodium citrate | 0.1332 g |
| Lemon roller dried | 0.0600 g |
| Lemon flavour 1 | 0.2000 g |
| Aspartame | 0.0700 g |
| Curcumin | 0.0032 g |

| | |
|---|---|
| Gingerin (0.1% in ethanol) | 0.0400 g |
| Magnesium gluconate | 0.4000 g |
| Thaumatin (0.1% soln) | 0.3000 g |
| Glycine | 0.1600 g |
| Soluble starch | 0.1000 g |

The above composition was added to water to provide a solution having a total volume of 40 ml. The composition was heated to 60°C and was found to have a pleasant taste with the bitterness of the paracetamol effectively masked.

### Control Example 1

A control composition was prepared by mixing together the following components in the amounts indicated:

| | |
|---|---|
| Paracetamol | 0.6500 g |
| Ascorbic acid | 0.0500 g |
| Caster sugar | 3.5000 g |
| Citric acid | 0.1868 g |
| Sodium citrate | 0.1332 g |
| Lemon roller dried | 0.0600 g |
| Lemon flavour 1 | 0.2000 g |
| Aspartame | 0.0700 g |
| Curcumin | 0.0032 g |

The above composition was added to water to provide a solution having a total volume of 40ml. The composition was heated to 60°C and was found to have a bitter taste. Further control formulations were prepared in a similar manner except for varying the amount of paracetamol. A composition containing 40% of the above amount of paracetamol was found to be only slightly bitter. A composition containing 60% of the paracetamol was found to have about one third of the bitterness of the 100% control, and a composition containing 80% of the paracetamol was found to have about two thirds of the bitterness of the 100% control. The bitterness is not directly proportional to the amount of paracetamol since the formulation already contains sodium citrate which acts to reduce some of the bitterness of the paracetamol. It is only when the amount of paracetamol exceeds the inhibiting capacity of the sodium citrate that bitterness is perceived.

### Comparative Example 1

To the formulation of Control Example 1 having a total volume of 40ml and containing 100% paracetamol were added various amounts of gingerin, ginger oil and soluble starch. The compositions were evaluated for bitterness masking using the scale defined in Control Example 1.

**TABLE I**

| **Additive** | **Concentration (g/serving)** | **Equi-bitter paracetamol concentration (%)** |
|---|---|---|
| Gingerin (0.1% in ethanol) | 0.0050 | 100 |
| | 0.0100 | 95 |
| | 0.0200 | 90 |
| | 0.0400 | 80 (taste ginger) |
| | 0.0800 | 75 (strong ginger) |
| Ginger oil (0.1% in ethanol | 0.0050 | 100 |
| | 0.0100 | 100 |
| | 0.0200 | 90 |
| | 0.0400 | 80 (taste ginger) |
| | 0.0800 | 70 (taste ginger) |
| Instant starch (Ultratex 4) | 0.025 | 100 |
| | 0.050 | 95 |
| | 0.100 | 80-85 |
| | 0.200 | 80 |
| | 0.400 | 80 |

### COMPARATIVE EXAMPLE 2

To the formulation of Control Example 1 having a total volume of 40ml and containing 100% paracetamol were added various amounts of magnesium glucomate, Talin (Thaumatin), sucrose ester, glycine and sodium chloride. The compositions were evaluated for bitterness masking using the scale defined in Control Example 1.

**TABLE II**

| **Additive** | **Concentration (g/serving)** | **Equi-bitter paracetamol concentration (%)** |
|---|---|---|
| Magnesium gluconate | 0.10 | 90 |
| | 0.20 | 85 |
| | 0.40 | 80-82 |
| | 0.80 | 75 |
| | 1.60 | 70 |
| Talin (0.1% solution) | 0.10 | 100 |
| | 0.20 | 95-100 |
| | 0.30 | 90 |
| | 0.40 | 85-90 |
| | 0.50 | 80 |
| Sucrose ester | 0.025 | 100 |
| | 0.050 | 100 |
| | 0.100 | 95-100 |
| | 0.150 | 90-95 |
| | 0.200 | 90 |
| Glycine | 0.020 | 100 |
| | 0.040 | 95 |
| | 0.080 | 90 |
| | 0.160 | 85 |
| | 0.320 | 80 |
| Sodium chloride | 0.0025 | 100 |
| | 0.0050 | 100 |
| | 0.010 | Possible slight reduction |
| | 0.020 | Salty |
| | 0.040 | Salty |

### Example 2

To the formulation of Control Example 1 having a total volume of 40ml and containing 100% paracetamol were added various amounts of a combinations of components. The compositions were evaluated for bitterness masking using the scale defined in Control Example 1.

**TABLE III**

| Three-Compoment Blends (including the sodium citrate | | |
|---|---|---|
| **Additive** | **Concentration (g/serving)** | **Equi-bitter paracetamol concentration (%)** |
| Ginger oil (0.1% solution) | 0.08 | 70 |
| Starch | 0.10 | |
| Starch | 0.10 | 75 |
| Magnesium gluconate | 1.60 | |
| Magnesium gluconate | 0.60 | 70 |
| Talin (0.1% solution) | 0.50 | |
| Talin (0.1% solution) | 0.50 | 80 |
| Glycine | 0.32 | |
| Glycine | 0.08 | |
| Ginger oil (0.1% solution) | 0.32 | 80 |

| Four-Compartment Blends (including the sodium citrate) | | |
|---|---|---|
| **Additive** | **Concentration (g/serving)** | **Equi-bitter paracetamol concentration (%)** |
| Ginger oil (0.1% solution) | 0.08 | 75 |
| Starch | 0.10 | |
| Magnesium gluconate | 1.60 | |
| Starch | 0.10 | 65 |
| Magnesium gluconate | 1.60 | |
| Talin (0.1% solution) | 0.50 | |
| Magnesium gluconate | 1.60 | 65-70 |
| Talin (0.1% solution) | 0.50 | |
| Glycine | 0.32 | |
| Talin (0.1% solution) | 0.50 | 80 |
| Glycine | 0.32 | |
| Ginger oil | 0.08 | |
| Glycine | 0.32 | 80 |
| Ginger oil (0.1% solution) | 0.08 | |
| Starch | 0.10 | |
| Magnesium gluconate | 1.60 | 70 |
| Talin (0.1% solution) | 0.50 | |
| Ginger oil (0.1% solution) | 0.08 | |

| Five Component Blends (including the sodium citrate) | | |
|---|---|---|
| **Additive** | **Concentration (g/serving)** | **Equi-bitter paracetamol concentration (%)** |
| Ginger oil (0.1% solution) | 0.08 | 60 |
| Starch | 0.10 | |
| Magnesium gluconate | 1.60 | |
| Talin (0.1% solution) | 0.50 | |
| Ginger oil (0.1% solution) | 0.08 | 70 |
| Starch | 0.10 | |
| Magnesium gluconate | 1.60 | |
| Glycine | 0.32 | |
| Ginger oil (0.1% solution) | 0.08 | 75-80 |
| Magnesium gluconate | 1.60 | |
| Talin (0.1% solution) | 0.50 | |
| Glycine | 0.32 | |
| Talin (0.1% solution) | 0.50 | 65 |
| Glycine | 0.32 | |
| Ginger oil | 0.08 | |
| Starch | 0.10 | |
| Glycine | 0.32 | 75 |
| Magnesium gluconate | 1.60 | |
| Talin (0.1% solution) | 0.50 | |
| Glycine | 0.10 | |

| Six Component Blend (including the sodium citrate) | | |
|---|---|---|
| **Additive** | **Concentration (g/serving)** | **Equi-bitter paracetamol concentration (%)** |
| Ginger oil (0.1% solution) | 0.08 | 75 |
| Starch | 0.10 | |
| Magnesium gluconate | 1.60 | |
| Talin (0.1% solution) | 0.50 | |
| Glycine | 0.32 | |

### Comparative Example 3

The best performing blend from Example 2 was found to be:

| | |
|---|---|
| Ginger oil (0.1% solution) | 0.20% |
| Starch | 0.25% |
| Magnesium gluconate | 4.00% |
| Talin (0.1% solution) | 1.25% |

This blend was added to aqueous solutions or dispersions of other pharmaceutical compounds known to have a bitter taste, namely Diclofenac (0.017%), Naproxen (0.167%), and Ketoprofen (0.033%). These were then assessed for bitterness against the corresponding formulations which did not contain this blend.

There was found to be a marginal decrease in the bitterness of Diclofenac, and no change or even an increase in the bitterness of Naproxen and Ketoprofen.

### Example 3

To the formulation of Control Example 1 having a total volume of 40ml and containing 100% paracetamol were individually added the following edible emulsifiers in amounts of 0.1g per dose:
Sodium steroyl-1-lactylate HLB10
Sucrose ester HLB 14
Polysrbate 80 HLB 15

The formulations containing Polysorbate 80 or sucrose ester both had reduced bitterness, but there was no reduction in the bitterness of the formulation containing sodium steroyl-2-actylate, showing that a high HLB value is necessary for masking the bitterness of paracetamol.

## Claims

1. A pharmaceutical composition comprising paracetamol and a masking component which reduces or masks the bitter taste thereof which comprises at least two members chosen from the following groups i to vi:
i. pharmacologically acceptable magnesium salt, magnesium oxide and/or magnesium hydroxide;
ii. gingerin and/or ginger oil;
iii. a delayed onset long lasting sweetener;
iv. soluble starch in a weight ratio of at least 1:15 with respect to the paracetamol;
v. an edible emulsifier having an HLB of greater than 10; and
vi. glycine.

2. A composition according to claim 1 wherein component i comprises magnesium chloride, gluconate, hydroxide, carbonate or bicarbonate and/or a mixture of magnesium chloride and magnesium sulfate.

3. A composition according claim 1 or 2 wherein component ii comprises ginger oil.

4. A composition according to any one of the preceding claims wherein component iii comprises Thaumatin, Neohesperdine DC and/or Glycyrrhizin.

5. A composition according to any one of the preceding claims wherein component v comprises a sucrose ester.

6. A composition according to any one of the preceding claims wherein the masking component also comprises:
vii. a pharmacologically acceptable sodium salt.

7. A composition according to claim 6 wherein component vii comprises sodium acetate, carbonate, bicarbonate, citrate, gluconate, malate, lactate, chloride, propionate, oxalate, malonate, succinate, fumarate, tartrate and/or benzoate.

8. A composition according to any one of the preceding claims wherein component i is present in a molar ratio of from 0.0001:1 to 0.01:1 calculated as magnesium atoms: paracetamol.

9. A composition according to any one of the preceding claims wherein component ii is present in a weight ratio of from 1:20,000 to 1:10,000 with respect to the paracetamol.

10. A composition according to any one of the preceding claims wherein component iii is present in a weight ratio of from 1:20,000 to 1:100 with respect to the paracetamol.

11. A composition according to any one of the preceding claims wherein component iv is present in a weight ratio of from 1:10 to 1:1 with respect to paracetamol.

12. A composition according to any one of the preceding claims wherein component v is present in in a weight ratio of from 1:10 to 2:1 with respect to paracetamol.

13. A composition according to any one of the preceding claims wherein component vi is present in in a molar ratio of from 1:4 to 2:1 with respect to paracetamol.

14. A composition according to any one of the preceding claims wherein component vii is present in a molar ratio of from 0.01:1 to 1:1 calculated as sodium atoms:paracetamol.

15. A composition according to any one of the preceding claims which comprises at least three members selected from groups i to vi.

16. A composition according to claim 15 which comprises at least for members selected from groups i to vii.

17. A composition according to any one of the preceding claims which comprises at least two members selected from groups i and iv.

18. A composition according to any one of the preceding claims which is in unit dosage form.

19. A composition according to any one of the preceding claims which is in the form of an aqueous solution.

20. A composition according to claim 19 wherein the composition has a volume of less than or equal to 50ml.

21. A composition according to claim 19 or 20 which is packaged in a self-heating container.

22. A composition as defined in any one of the preceding claims for use in a method of treatment of the human or animal body by therapy.

23. A composition as defined in any one of claims 1 to 21 for use in a method of treatment of the symptoms of colds and influenza in humans.

24. Use of a masking component as defined in any one of claims 1 to 17 to reduce or mask the bitter taste of paracetamol.

25. Use of a masking component as defined in any one of claims 1 to 17 in the manufacture of a medicament comprising paracetamol for the treatment of the symptoms of colds and influenza in humans.

26. Use of gingerin, ginger oil, soluble starch or a mixture thereof to reduce or mask the bitter taste of paracetamol.

## Patentansprüche

1. Arzneimittel, umfassend Paracetamol und einen den bitteren Geschmack davon reduzierenden oder überdeckenden Markierungsbestandteil, der mindestens zwei Vertreter aus den folgenden Gruppen i. bis vi. umfasst:
i. pharmakologisch verträgliches Magnesiumsalz, Magnesiumoxid und/oder Magnesiumhydroxid,
ii. Gingerin und/oder Ingweröl,
iii. einen lang anhaltenden Süßstoff mit verzögertem Einsatz,
iv. lösliche Stärke in einem Gewichtsverhältnis von mindestens 1:15 in Bezug auf das Paracetamol,
v. einen essbaren Emulgator mit einem HLB-Wert von größer als 10 und
vi. Glycin.

2. Arzneimittel nach Anspruch 1, wobei Bestandteil i. Magnesiumchlorid gluconat, -hydroxid, -carbonat oder -bicarbonat und/oder ein Gemisch aus Magnesiumchlorid und Magnesiumsulfat umfasst.

3. Arzneimittel nach Anspruch 1 oder 2, wobei Bestandteil ii. Ingweröl umfasst.

4. Arzneimittel nach einem der vorangehenden Ansprüche, wobei Bestandteil ii. Thaumatin, Neohesperdin DC und/oder Glycyrrhizin umfasst.

5. Arzneimittel nach einem der vorangehenden Ansprüche, wobei Bestandteil v. einen Saccharosester umfasst.

6. Arzneimittel nach einem der vorangehenden Ansprüche, wobei der Überdeckungsbestandteil auch Folgendes umfasst:
vii. ein pharmakologisch verträgliches Natriumsalz.

7. Arzneimittel nach Anspruch 6, wobei Bestandteil vii. Natriumacetat, -carbonat, -bicarbonat, -citrat, -gluconat, -malat, -lactat, -chlorid, -propionat, -oxalat, -malonat, -succinat, -fumarat, -tartrat und/oder -benzoat umfasst.

8. Arzneimittel nach einem der vorangehenden Ansprüche, wobei Bestandteil i. in einem Molverhältnis von 0,0001:1 bis 0,01:1, berechnet als Magnesiumatome:Paracetamol, vorliegt.

9. Arzneimittel nach einem der vorangehenden Ansprüche, wobei Bestandteil ii. in einem Molverhältnis von 1:20.000 bis 1:10.000 in Bezug auf Paracetamol vorliegt.

10. Arzneimittel nach einem der vorangehenden Ansprüche, wobei Bestandteil iii. in einem Molverhältnis von 1:20.000 bis 1:100 in Bezug auf Paracetamol vorliegt.

11. Arzneimittel nach einem der vorangehenden Ansprüche, wobei Bestandteil iv. in einem Molverhältnis von 1:10 bis 1:1 in Bezug auf Paracetamol vorliegt.

12. Arzneimittel nach einem der vorangehenden Ansprüche, wobei Bestandteil v. in einem Molverhältnis von 1:10 bis 2:1 in Bezug auf Paracetamol vorliegt.

13. Arzneimittel nach einem der vorangehenden Ansprüche, wobei Bestandteil vi. in einem Molverhältnis von 1:4 bis 2:1 in Bezug auf Paracetamol vorliegt.

14. Arzneimittel nach einem der vorangehenden Ansprüche, wobei Bestandteil vii. in einem Molverhältnis von 0,01:1 bis 1:1 in Bezug auf Paracetamol vorliegt.

15. Arzneimittel nach einem der vorangehenden Ansprüche, das mindestens drei Vertreter, ausgewählt aus i. bis vi., umfasst.

16. Arzneimittel nach Anspruch 15, das mindestens vier Vertreter, ausgewählt aus i. bis vii., umfasst.

17. Arzneimittel nach einem der vorangehenden Ansprüche, das mindestens zwei Vertreter, ausgewählt aus i. bis iv., umfasst.

18. Arzneimittel nach einem der vorangehenden Ansprüche, das in Dosierungseinheitsform vorliegt.

19. Arzneimittel nach einem der vorangehenden Ansprüche, das in Form einer wässrigen Lösung vorliegt.

20. Arzneimittel nach Anspruch 19, wobei das Arzneimittel ein Volumen von weniger als oder gleich 50 ml aufweist.

21. Zusammensetzung nach Anspruch 19 oder 20, das in einem selbst erwärmenden Behälter verpackt ist.

22. Zusammensetzung, definiert in einem der vorangehenden Ansprüche, zur Verwendung in einem Verfahren zur Behandlung des menschlichen oder tierischen Körpers durch Therapie.

23. Arzneimittel, definiert in einem der Ansprüche 1 bis 21, zur Verwendung in einem Verfahren zur Behandlung der Symptome von Erkältungen und Grippe bei Menschen.

24. Verwendung eines Maskierungsbestandteils, definiert in einem der Ansprüche 1 bis 17, zum Reduzieren oder Überdecken des bitteren Geschmacks von Paracetamol.

25. Verwendung eines Maskierungsbestandteils, definiert in einem der Ansprüche 1 bis 17, bei der Herstellung eines Paracetamol umfassenden Medikaments zur Behandlung der Symptome von Erkältungen und Grippe bei Menschen.

26. Verwendung von Gingerin, Ingweröl, löslicher Stärke oder eines Gemischs davon zum Reduzieren oder Überdecken des bitteren Geschmacks von Paracetamol.

## Revendications

1. Composition pharmaceutique comprenant du paracétamol et un constituant de masquage qui réduit ou masque son goût amer qui comprend au moins deux membres choisis parmi les groupes i à vi suivants :
i. un sel de magnésium, oxyde et/ou hydroxyde de magnésium pharmacologiquement acceptable ;
ii. la gingérine et/ou l'essence de gingembre ;
iii. un édulcorant à effet retard et à longue durée d'action ;
iv. un amidon soluble en un rapport pondéral d'au moins 1:15 par rapport au paracétamol ;
v. un émulsifiant comestible ayant une valeur d'équilibre hydrophile-lipophile supérieure à 10 ; et
vi. la glycine.

2. Composition suivant la revendication 1, dans laquelle le constituant i comprend le chlorure, gluconate, hydroxyde, carbonate ou bicarbonate de magnésium et/ou un mélange de chlorure de magnésium et de sulfate de magnésium.

3. Composition suivant la revendication 1 ou 2, dans laquelle le constituant ii comprend l'essence de gingembre.

4. Composition selon l'une quelconque des revendications précédentes, dans laquelle le constituant iii comprend la thaumatine, la néohespéridine DC et/ou la glycyrrhizine.

5. Composition suivant l'une quelconque des revendications précédentes, dans laquelle le constituant v comprend un ester de saccharose.

6. Composition suivant l'une quelconque des revendications précédentes, dans laquelle le constituant de masquage comprend également :
vii. un sel de sodium pharmacologiquement acceptable.

7. Composition suivant la revendication 6, dans laquelle le constituant vii comprend l'acétate, le carbonate, le bicarbonate, le citrate, le gluconate, le malate, le lactate, le chlorure, le propionate, l'oxalate, le malonate, le succinate, le fumarate, le tartrate et/ou le benzoate de sodium.

8. Composition suivant l'une quelconque des revendications précédentes, dans laquelle le constituant i est présent en un rapport molaire de 0,0001:1 à 0,01:1 calculé en atomes de magnésium:paracétamol.

9. Composition suivant l'une quelconque des revendications précédentes, dans laquelle le constituant ii est présent en un rapport pondéral de 1:20 000 à 1:10 000 par rapport au paracétamol.

10. Composition suivant l'une quelconque des revendications précédentes, dans laquelle le constituant iii est présent en un rapport pondéral de 1:20 000 à 1:100 par rapport au paracétamol.

11. Composition suivant l'une quelconque des revendications précédentes, dans laquelle le constituant iv est présent en un rapport pondéral de 1:10 à 1:1 par rapport au paracétamol.

12. Composition suivant l'une quelconque des revendications précédentes, dans laquelle le constituant v est présent en un rapport pondéral de 1:10 à 2:1 par rapport au paracétamol.

13. Composition suivant l'une quelconque des revendications précédentes, dans laquelle le constituant vi est présent en un rapport molaire de 1:4 à 2:1 par rapport au paracétamol.

14. Composition suivant l'une quelconque des revendications précédentes, dans laquelle le constituant vii est présent en un rapport molaire de 0,01:1 à 1:1 calculé en atomes de sodium:paracétamol.

15. Composition suivant l'une quelconque des revendications précédentes, qui comprend au moins trois membres choisis parmi les groupes i à vi.

16. Composition suivant la revendication 15, qui comprend au moins quatre membres choisis parmi les groupes i à vii.

17. Composition suivant l'une quelconque des revendications précédentes, qui comprend au moins deux membres choisis parmi les groupes i et iv.

18. Composition suivant l'une quelconque des revendications précédentes, qui est sous une forme posologique unitaire.

19. Composition suivant l'une quelconque des revendications précédentes, qui est sous forme d'une solution aqueuse.

20. Composition suivant la revendication 19, ladite composition ayant un volume inférieur ou égal à 50 ml.

21. Composition suivant la revendication 19 ou 20, qui est emballée dans un récipient auto-chauffant.

22. Composition telle que définie dans l'une quelconque des revendications précédentes, destinée à être utilisée dans une méthode de traitement de l'organisme de l'homme ou d'un animal par thérapie.

23. Composition telle que définie dans l'une quelconque des revendications 1 à 21, destinée à être utilisée dans une méthode de traitement des symptômes du rhume et de la grippe chez l'homme.

24. Utilisation d'un constituant de masquage tel que défini dans l'une quelconque des revendications 1 à 17, pour réduire ou masquer le goût amer du paracétamol.

25. Utilisation d'un constituant de masquage tel que défini dans l'une quelconque des revendications 1 à 17, dans la production d'un médicament comprenant du paracétamol pour le traitement des symptômes du rhume et de la grippe chez l'homme.

26. Utilisation de gingérine, d'essence de gingembre, d'amidon soluble ou d'un mélange de ceux-ci pour réduire ou masquer le goût amer du paracétamol.
